Europäisches Patentamt

European Patent Office

Office européen des brevets

(11) Publication number: **0 067 803**
**B1**

(12) **EUROPEAN PATENT SPECIFICATION**

(45) Date of publication of patent specification: **24.09.86**

(21) Application number: **82850133.8**

(22) Date of filing: **11.06.82**

(51) Int. Cl.⁴: **A 61 M 16/01**

(54) Pneumatic control unit for breathing apparatus.

(30) Priority: **12.06.81 SE 8103695**

(43) Date of publication of application:
**22.12.82 Bulletin 82/51**

(45) Publication of the grant of the patent:
**24.09.86 Bulletin 86/39**

(84) Designated Contracting States:
**AT BE CH DE FR GB IT LI LU NL**

(56) References cited:
**DE-A-2 735 555**
**DE-C- 929 637**
**US-A-3 621 842**

(73) Proprietor: **Mediplast AB**
**Rasundavägen 60**
**S-171 52 Solna (SE)**

(72) Inventor: **Jacob, Peter**
**Violvägen 6**
**S-810 60 Söderfors (SE)**

(74) Representative: **Roth, Ernst Adolf Michael et al**
**GÖTEBORGS PATENTBYRA AB Box 5005**
**S-402 21 Göteborg (SE)**

Courier Press, Leamington Spa, England.

## Description

The present invention relates to a breathing apparatus for administration of breathing gas in for example surgical operations, in which the breathing gases from a patient are instantaneously stored in an elastic breathing bag, the bag being provided in a rigid pressure chamber, the inner space of which being adjustable in relation to its volume, whereby a pneumatic control unit is provided to control the insufflation pressure in the pressure chamber intermittently and via an injector, see DE—C—929 637.

In surgical operations it is normally required that the patient is anaesthetized and is remained in this condition during the whole operation. This is achieved by supplying anaesthetics to the patient. The anaesthetics can be administered intraveniously directly to the vains of the patient or by letting the patient breath anaesthetics in a gaseous condition, so called inhalation anaesthetics. Depending on the kind of the surgical operation the duration of the narcosis can be more or less long and thus a careful control of the narcosis procedure is necessary. In this connection it is necessary to be able to control the breathing gas, especially its composition of oxygen, anaesthetic gas, humidity and temperature. The anaesthetic apparatuses and the breathing apparatuses nowadays in use suffer from several drawbacks. Besides the fact that the apparatuses are expensive they are relatively difficult to handle and supervise. Several value means and reducing valves as well as electrical functions require adjustment and supervision. This also requires unceasing observation during the operation.

Since the hygienic requirement in these connections are very high it is a requirement that after each operation a careful cleaning and sterilization is effected, which among others implies that the apparatus must be disassembled and must be cleaned. This requires a lot of time besides the fact that the cleaning must be effected by a skilled staff. As a result hereof the operation of such apparatuses is relatively cost consuming.

In the DE—A1—2735555, a control unit is disclosed for an apparatus which control the supply of gas to a patient. The gas to the patient flow through the control unit. This apparatus does not use any of the expiration gases from the patient. In the DE—C—929637, a breathing apparatus is disclosed wherein the change between the inspiration and expiration phase are controlled by the pressure in the chamber (11). The length of the breathing phases can therefore not immediately be determined.

The purpose of the present invention is thus to eliminate the drawbacks mentioned above and to provide a pneumatic control unit for a breathing apparatus by which each breathing phase—the inspiration and the expiration phase—can be individually adjusted. The breathing apparatus containing the pneumatic control unit can be manufactured to low costs and is easy to maintain. The characteristics of the invention are described in the claim.

The invention will be described below by way of an example, reference being made to the accompanying drawings, on which

Fig. 1 is a schematic view of a breathing apparatus, and

Fig. 2 is an elementary view of a pneumatic control unit for controlling the breathing apparatus shown in Fig. 1.

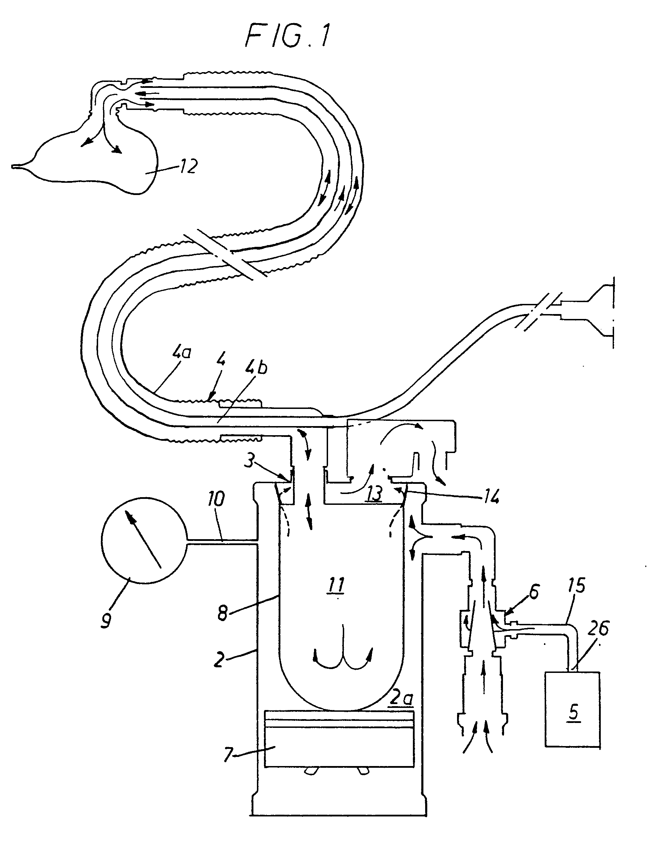

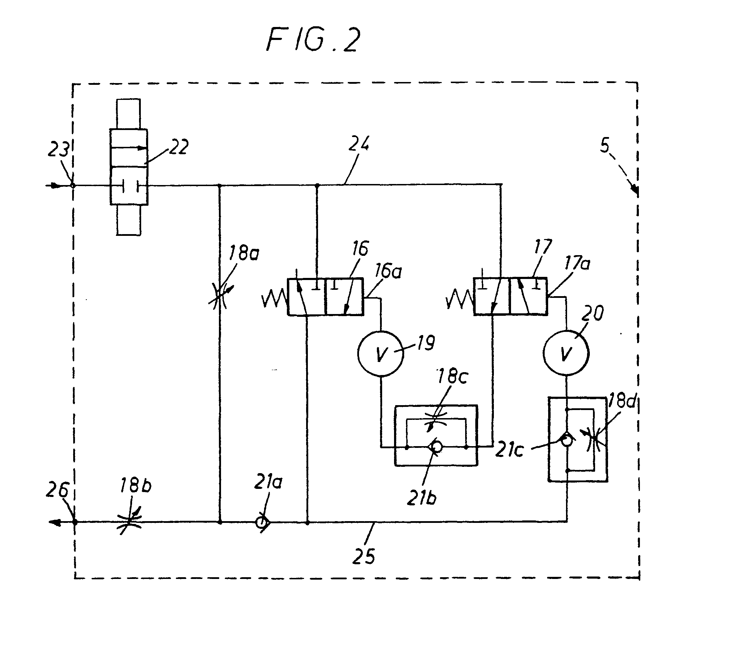

In Fig. 1 there is shown a breathing apparatus for controlling breathing gas to a patient, especially a narcosis apparatus for so called partial rebreathing without $CO_2$-absorption of the type Mapleson D comprising a rigid container forming a pressure chamber 2, an excess valve 3 comprising an elastic breathing bag 8 provided therein and a flexible coaxial tube 4 provided between the patient (not shown) and the breathing apparatus. For controlling the gas flow to and from the patient, that is the control of the gas pressure and the breathing rate, a pneumatic control unit 5 (see Fig. 2) is provided and is connected to the inner space 2a of the pressure chamber 2 via an operating unit, i.e. an injector 6. The pressure chamber 2 is provided with a moveable bottom 7 for adjusting the volume of the space 2a. By this the volume of the breathing gas, i.e. the tidal volume, in the excess valve 3 can be predetermined or can be controlled. The tidal volume can for example amount to 1.000 ml for adults and 400 ml for children. For supervision and control of the working pressure, i.e. the insufflation pressure, prevailing in the inner space 2a of the pressure chamber 2 a pneumatic manometer 9 is provided which via a conduit 10 is connected to the pressure chamber 2. Fresh gas, consisting for example of anaesthetic gas mixed with oxygen or air, is supplied to the patient via the central tube 4b of the coaxial tube 4, the outer part 4a of which being in communication with the first space 11 of the excess valve 3 which is enclosed by the elastic breathing bag 8 made for example of rubber. A breathing mask 12 preferably made of electrically conducting rubber is provided in the other end of the coaxial tube 4.

Besides a first space 11 the excess valve 3 is provided with a second space 13 communicating with the atmosphere via an exhausting device. By the construction of the excess valve 3 with the breathing bag 8 applied outside the sidewall 14 of the second space 13 in which wall openings (not shown) are provided, the breathing out air from the patient to said first space 11 can be passed off to said second space 13 and the further out into the atmosphere via an exhausting device. The breathing bag 8 expands when the gas pressure increases from the inside thereof and when this pressure has become sufficiently large the breathing bag 8 loses its sealing effect against the sidewall 14 of said second space 13, the openings being free and the breathing air being able to pass from the inner space 11 of the breathing bag 8 to the second space 13 of the excess valve (confer the dotted lines in Fig. 1).

By supplying compressed air via a pneumatic conduit 15 by an injector 6 to the pressure chamber 2 a stream of air flows from the surrounding atmosphere into the pressure chamber 2, a positive pressure being formed in the inner space 2a of the pressure chamber. This working pressure or this insufflation pressure compresses the breathing bag 8, its contents of breathing gas being pressed back to the patient via the coaxial tube 4. The gas supplied in this way to the patient has an appropriate temperature and humidity while it is admixed with anaesthetic gas and oxygen which is supplied closed to the breathing mask 12 via the inner tube 4b. This inner tube 4b is for example connected to an adjustable gas mixture apparatus (not shown) or to a gas output provided close to the breathing apparatus.

For controlling the insufflation pressure the pneumatic control unit 5 is provided to supply the injector 6 with gas under pressure. The pneumatic control unit 5 comprises a first and a second pneumatically controlled main valve 16, 17, four throttle valves 18a—18d, a first and a second volume 19, 20, three non-return valves 21a—21c and a two way valve 22 which can be manually actuated. Compressed air is supplied to the input 23 of the control unit 5, and passes the two way valve 22 by which the control unit 5 is turned on and off. A first throttle valve 18a permits a basic flow of air to pass from the feedline 24 to the backline 25 and creates a basic pressure at the output 26 of the control unit 5 which is the PEEP (Positive Endexpiratory Pressure). Through a second throttle valve 18b the working pressure or the insufflation pressure is set or adjusted which throttle valve 18b thus delimits the pressure of the air supplied via the output 26 to the injector 6. Besides the first throttle valve 18a the first and the second main valves 16, 17 are connected to the feed-line 24 of the control unit 5. The first main valve 16 is normally closed and the second main valve 17 is normally opened. A first and a second volume 19, 20 are connected to the control connections 16a, 17a of the main valves 16, 17 which volumes function to create a switching in delay and a switching off delay from the time when air begins to flow via the throttle valves 18c, 18d connected in parallel with the non-return valves 21b, 21c until the main valves 16, 17 are actuated. Usually the main valves 16, 17 function in such a way that they are actuated at 75% of the pneumatic working pressure and are reversed at 25%.

The pneumatic unit 5 operates in a cyclic way with an inspiration phase and an expiration phase. Their lengths can be adjusted independent of each other via the throttle valves 18d and 18c respectively. A proper breathing rate in using a partial rebreathing system without $CO_2$-absorption is thirteen breaths per minute and this basic level is marked at the throttle valves 18c, 18d (not shown). Compressed air is thus fed via the input 23 to the two-way valve 22 which in its actuated position permits the air to pass further into the conduit 24. The other main valve 17 which in this phase, i.e. the expiration phase, is open permits air to pass to the first volume 19 via the throttle valve 18c. When the required reversing pressure has been achieved in the volume 19 the first main valve 16 is actuated, air flowing into the conduit 25 and further via the non-return valve 21a and the throttle valve 18b to the output of the control unit 5. After that the inspiration phase begins. The other volume 20 is supplied with compressed air via the conduit 25 and the throttle valve 18d. When the required reversing pressure has been achieved the other main valve 17 is actuated and the pressure of the first volume 19 is unloaded via the non-return valve 21b. This results in the fact that the first main valve 16 is reset to its inactivated position and the expiration phase begins again. In the other volume 20 the pressure is now reduced via the non-return valve 21c as a result of the fact that the pressure at the output 26 of the control unit 5 is lower than the pressure in the volume 20. This procedure is repeated in a cyclic way as long as the two-way valve 22 is open.

The invention is not restricted to the example of the embodiment described above but several alternative embodiments are possible within the scope of the claims.

## Claim

A breathing apparatus for administration of breathing gas in for example surgical operations, in which the breathing gases from a patient are instantaneously stored in an elastic breathing bag (8), the bag (8) being provided in a rigid pressure chamber (2), the inner space (2a) of which being adjustable in relation to its volume, whereby a pneumatic control unit (5) is provided to control the insufflation pressure in the pressure chamber (2) intermittently and via an injector (6), characterised in that, the control unit (5) comprises a first main valve (16) pneumatically controlled by a second main valve (17) via a first storage volume (19) and a first adjustable throttle valve (18c) which is connected in parallel to a non-return valve (21b), and the second main valve (17) pneumatically controlled by the first main valve (16) via a second storage volume (20) and a second adjustable throttle valve (18d) which is connected in parallel to a second non-return valve (21c), further characterised in that the first main valve (16) is connected to the injector (6) via a third throttle valve (18b) and characterised in that the first throttle valve (18c) and the first storage volume (19) are provided to control the length of the inspiration phase, whereas the second throttle valve (17) and the second storage volume (20) are provided to control the length of the expiration phase.

## Patentanspruch

Atemgerät zur Administration von Atmungsgas bei beispielsweise chirurgischen Eingriffen, bei dem die Atmungsgase eines Patienten unverzögert in einem elastischen Atmungsbeutel (8) gespeichert werden, welcher Beuteil (8) in einer starren Druckkammer (2) angeordnet ist, deren Innenraum (2a) bezüglich seines Volumens veränderbar ist, wobei eine pneumatische Steuereinheit (5) zum intermittierenden Steuern des Einblasedruckes und mittels eines Injektors (6) vorhanden ist, dadurch gekennzeichnet, dass die Steuereinheit (5) ein erstes Hauptventil (16) aufweist, das von einem zweiten Hauptventil (17) überein erstes Speichervolumen (19) und einem ersten verstellbaren Steuerventil (18c), das parallel zu einem Rückschlagventil (21b) geschaltet ist, pneumatisch gesteuert ist, und das zweite Hauptventil (17) vom ersten Hauptventil (16) über ein zweites speichervolumen (20) und einem zweiten verstellbaren Steuerventil (18d), das parallel zu einem zweiten Rückschlagventil (21c) geschaltet ist, pneumatisch gesteuert ist, dass das erste Hauptventil (16) über ein drittes Steuerventil (18b) mit dem Injektor (6) verbunden ist, und das das erste Steuerventil (18c) und das erste Speichervolumen (19) zum Steuern der Dauer der Einatmungsphase dienen, wohingegen das zweite Hauptventil (17) und das zweite Speichervolumen (20) zum Steuern der Dauer der Ausatmungsphase dienen.

## Revendication

Appareil respiratoire pour l'administration de gaz respiratoire, par exemple pour les opérations chirurgicales, dans lequel les gaz respiratoires du patient sont instantanément stockés dans un sac respiratoire élastique (8), le sac (8) étant monté à l'intérieur d'une chambre de pression rigide (2) l'espace interne (2a) de celle-ci étant réglable par rapport à son volume et dans lequel une unité de commande pneumatique (5) est prévue pour commander par intermittence la pression d'insufflation dans la chambre de pression (2) par un dispositif d'injection (6) caractérisé en ce que, l'unité de commande (5) comprend une première soupape principale (16) commandée pneumatiquement par une seconde soupape principale (17) à travers un premier volume de stockage (19) et une première soupape d'étranglement réglable (18c) qui est reliée, en parallèle à une soupape anti-retour (21b), et la seconde soupage principale (17) est commandée pneumatiquement par la première soupage principale (16) à travers un second volume de stockage (20) et une seconde soupape d'étranglement réglable (18d) qui est reliée en parallèle à une seconde soupape anti-retour (21c), caractérisé en outre en ce que la première soupape principale (16) est reliée au dispositif d'injection (6) par l'intermédiaire d'une troisième soupape d'étranglement (18b) et en ce que la première soupape d'étranglement (18c) et le premier volume de stockage (19) sont destinés à commander la longueur de la phase d'inspiration, alors que la seconde soupape d'étranglement (17) et le second volume de stockage (20) sont destinés à commander la longueur de la phase d'expiration.

FIG. 1

# FIG.2